# EUROPEAN PATENT APPLICATION

(11) **EP 2 409 684 A1**
(43) Date of publication of application: **25.01.2012**
(21) Application number: 10753502.3
(22) Date of filing: 16.03.2010
(51) Int. Cl.: A61K 8/898, A61K 8/25, A61K 8/37, A61Q 1/12

(54) **PROCESS FOR PRODUCING POWDERY COMPOSITION AND POWDERY COSMETIC**

(30) Priority: 18.03.2009 JP 2009066861
(71) Applicant: Shiseido Company, Ltd., Chuo-ku Tokyo 104-8010 (JP)
(72) Inventor: KURAHASHI, Takuma, Yokohama-shi Kanagawa 224-8558 (JP); HATA, Hideo, Yokohama-shi Kanagawa 224-8558 (JP); OGURA, Yoshito, Yokohama-shi Kanagawa 224-8558 (JP)
(74) Representative: Henkel, Breuer & Partner
(86) International application number: PCT/JP2010/054383
(87) International publication number: WO 2010/107010

(57) **Abstract**

Disclosed is a process for producing a powdery cosmetic having an excellent feeling upon application, an excellent long-lasting property and excellent production efficiency. Specifically disclosed is a process for producing a powdery composition, which comprises: a slurry preparation step of mixing a powder component with a non-volatile oily component in a volatile solvent to prepare a slurry, wherein the non-volatile oily component comprises (A) an amino-modified silicone having a functional group equivalent of 1000 to 10, 000 and a viscosity of 200 to 2000 mm²/s at 25°C and (B) a lipophilic nonionic surfactant; and a drying step of drying the slurry to produce the powdery composition, wherein a drying apparatus to be used for the drying step can dry the slurry in such a manner that the slurry is divided into fine droplets by means of a mechanical shearing force and a drying gas is blown to the fine droplets. Also specifically disclosed is a powdery cosmetic having the above-mentioned properties, which is prepared from a powdery composition produced by the process.

## Description

### TECHNICAL FIELD

The present invention relates to a method for producing a powdery composition, a powdery cosmetic material prepared by incorporating the powdery composition obtained by the production method, and a method for producing a solid powdery cosmetic material using the powdery composition obtained by the production method. More particularly, the present invention relates to a method for producing a powdery makeup product such as a foundation or a makeup base, which has an excellent feeling of use and provides excellent long-lasting makeup.

### BACKGROUND ART

Along with the recent diversification and advances in consumers' needs, there has been a demand for a further improvement in powdery cosmetic materials, as represented by powdery foundation, not only in terms of usage properties such as appliability with a puff, or a satisfactory feeling of use when the cosmetic material is applied on the skin, but also in terms of productivity and the work environment. In response to these needs, a method for producing a powdery cosmetic material has been suggested, in which a powder component and an oily component are highly dispersed in a volatile solvent, and then the dispersion is dried into fine particles with a flash dryer (Patent Document 1).

The powdery cosmetic material obtained according to the production method is such that while the feeling of use when applied on the skin has a moist feeling like a liquid, a satisfactory texture of being soft dry and uniform is obtained. However, since the powder is uniformly coated with the oily component by slurring mixing in a volatile solvent, after the cosmetic material is applied, the cosmetic material is likely to be blended in with the sebum secreted from the skin, and the cosmetic material is not sufficiently satisfactory in terms of long-lasting makeup. Therefore, even though the cosmetic material can be provided as a powdery cosmetic material for autumn and winter seasons, it has been found difficult to apply the cosmetic material to a powdery cosmetic material for spring and summer seasons.

Furthermore, the production method described above has a problem that when a powder which undergoes a large color change when wetted with an oily component is incorporated, the powdery cosmetic material becomes dark by wetting, and therefore, the skin wearing makeup turns markedly dull due to sebum. Thus, even though the powdery cosmetic material has a good feeling of use, it has been difficult to incorporate the cosmetic material in large amounts.

In order to enhance the effect of long-lasting makeup in the production method described above, there have been proposed a technology of using a fluoridized powder in combination (Patent Document 2), and a technology of using a high viscosity and non-polar silicone in combination (Patent Document 3). However, in the case of using a fluoridized powder in combination, though the effect of long-lasting makeup is enhanced, since it is difficult for the fluoridized powder to blend in with the oily component, localization of the oily component is prone to occur during the drying step after the powder component is turned into slurry, fluctuation of the product quality is likely to occur, and a problem is posed in stable production of large amounts of the product. On the other hand, in the case of using high viscosity silicone in combination, if the high viscosity silicone has poor compatibility with a volatile solvent, aggregation is prone to occur, similarly fluctuation of the product quality is likely to occur, and a problem is posed in stable production of large amounts of the product.

Meanwhile, it is known to incorporate a highly polymerized amino-modified silicone into a makeup cosmetic material so as to enhance the long-lasting properties of makeup (Patent Document 4). However, the highly polymerized amino-modified silicone does not have fluidity, and it is necessary to use the amino-modified silicone in the form of dissolving in a volatile solvent or the like. Furthermore, the degree of freedom for the formulation or the container is low, and the film of the highly polymerized amino-modified silicone formed after the solvent volatilizes causes the effect of long-lasting makeup. Therefore, when the cosmetic material is applied to a powdery cosmetic material, the cosmetic material is not sufficiently satisfactory in terms of the feeling of use.

### PRIOR ART DOCUMENTS

### PATENT DOCUMENT

Patent Document 1: JP-A No. 2007-55990
Patent Document 2: JP-A No. 2008-189642
Patent Document 3: JP-A No. 2008-214267
Patent Document 4: JP-A No. Hei 5-32527

### SLTMMARY OF INVENTION

### PROBLEM TO BE SOLVED BY THE INVENTION

It is an object of the present invention to provide a production method which improves the product quality of a powdery cosmetic material and ameliorates productivity in the production process. It is another object of the present invention to provide a powdery cosmetic material, particularly, a powdery makeup cosmetic material such as foundation, which has an excellent moist feeling of use and provides excellent long-lasting makeup (preventing makeup smudging).

### MEANS FOR SOLVING PROBLEM

The present invention provides a method for producing a powdery composition, the method comprising:
a slurry preparation step of mixing a powder component and a non-volatile oily component in a volatile solvent to obtain a slurry; and
a drying step of drying the slurry to remove the volatile solvent,
wherein in the drying step, a drying apparatus which performs drying of the slurry by converting the slurry into fine liquid droplets by a mechanical shear force and blowing a dry gas to the fine liquid droplets, and
the non-volatile oily component contains:
   (a) an amino-modified silicone having a functional group equivalent of 1000 to 10,000 and a viscosity at 25°C of 200 to 2000 mm²/s, in an amount of 0.1% to 2% by mass relative to the total amount of the powdery composition, and
   (b) a lipophilic nonionic surfactant in an amount of 0.1% to 2% by mass relative to the total amount of the powdery composition.

The present invention provides a method for producing a powdery composition such as described above, wherein the drying apparatus used in the drying step is a drying apparatus including:
a hollow-shaped housing;
a shearing means that shears the slurry by a shearing member provided inside the housing and converts the slurry into fine liquid droplets;
a supplying means that supplies the slurry to the shearing member in the housing;
a blowing means that blows a dry gas into the housing, and supplying the drying gas to the slurry that has been converted to fine liquid droplets by the shearing means to bring the dry gas and the fine liquid droplets into contact; and
a collecting means that collects a powdery composition produced by drying the slurry.

The present invention provides a method for producing a powdery composition such as described above, wherein in the slurry preparation step, the slurry is obtained by mixing the powder component and the non-volatile oily component in the volatile solvent using a medium agitating mill, and cracking and/or pulverizing and/or dispersing the powder component.

Furthermore, there is provided a method for producing a powdery composition such as described above, wherein as the powder component and the non-volatile oily component, the powder component is contained in an amount of 70% to 98% by mass, and preferably 80% to 95% by mass, relative to the total amount of the powdery composition, and the non-volatile oily component is contained in an amount of 2% to 30% by mass, and preferably 5% to 20% by mass, relative to the total amount of the powdery composition.

There is also provided a method for producing a powdery composition such as described above, wherein a sorbitan fatty acid ester is contained as the lipophilic nonionic surfactant.

There is provided a method for producing a powdery composition such as described above, wherein the powder component further contains an extender pigment, a white pigment, and a color pigment.

There is provided a method for producing a powdery composition such as described above, wherein the powder component further contains red synthetic fluorphlogopite which contains iron atoms in the crystal structure, in an amount of 2% to 20% by mass.

Furthermore, the present invention provides a powdery cosmetic material containing the powdery composition obtained by the production method described above, in an amount of 80% to 100% relative to the total amount of the powdery cosmetic material.

The present invention also provides a method for producing a solid powdery cosmetic material, including a solidification step of filling the powdery composition obtained by any one of the production methods in a container, optionally together with other optional components such as a pearl pigment, and solidifying the powdery composition and the optional components by dry press molding, and a solid powdery cosmetic material obtainable by the production method.

### EFFECT OF THE INVENTION

According to the present invention, a powdery composition in which the powdery component does not aggregate even in the drying step, and the powdery component is uniformly coated with the non-volatile oily component and is imparted with water-repellent and oil-repellent effects, can be obtained. When this powdery composition is used, a powdery cosmetic material which has an excellent feeling of use, particularly, excellent adhesion to the skin, and an excellent moist feeling, and provides excellent long-lasting makeup, can be provided.

Furthermore, when the powdery composition is filled in a container and solidified, a solid powdery cosmetic material which can be very easily applied, and has an excellent feeling of use, and provides excellent long-lasting makeup, can be obtained.

According to the present invention, localization of the oily component does not easily occur even in the drying step, and a powdery cosmetic material having a uniform product quality can be stably produced even under mass production.

Furthermore, according to the present invention, even if a powder such as red synthetic fluorphlogopite which contains iron atoms in the crystal structure and undergoes a large color change when wetted with an oily component, is incorporated, dulling of color over time can be mitigated.

### BRIEF DESCRIPTION OF DRAWINGS

FIG. 1 is a schematic configuration diagram of the apparatus used in an embodiment of the present invention;
FIG. 2 is a diagram showing an example of the medium agitating mill; and
FIG. 3 is a diagram showing an example of the medium agitating mill.

### MODES FOR CARRYING OUT THE INVENTION

The present invention relates to a method for producing a powdery composition, which includes a slurry preparation step of mixing a powder component and a non-volatile oily component in a volatile solvent to obtain a slurry, and a particular drying step of obtaining the powder composition with the volatile solvent removed from the slurry. The inventors of the present invention particularly paid attention to a step of removing a volatile solvent in a so-called wet production method, by which a powdery composition is produced from a slurry containing a volatile solvent, and conducted a thorough investigation on various drying processes.

As a result, in an agitating drying machine or a vibrating drying machine, which is usually represented by a reduced pressure kneader, the powder particles are strongly aggregated by agitation during the drying process, and cracking with a pulverizer or the like is very insufficient. On the other hand, drying with a drying machine of a type called drum dryer or a spray dryer has also been taken into consideration, but aggregation occurs at the time of drying in all of these drying machines. Therefore, in order to obtain a powdery cosmetic material, cracking with a dry pulverizing machine such as a pulverizer is essential. In addition, since the drying efficiency is markedly low in conventional shelf-type drying machines, the productivity is poor from the viewpoint of the ability for mass production. Furthermore, spray dryers have a problem that the rate of drying process is markedly low, and when mass production is intended, an increase in the size of the apparatuses is unavoidable.

The inventors of the present invention have conducted a thorough investigation on such problems, and as a result, they found that when a drying apparatus which has a shearing mechanism of converting a slurry into fine liquid droplets, and is capable of drying the slurry in the form of fine liquid droplets is used, the resulting powdery composition is finely divided so that cracking with a dry pulverizing machine is not needed.

The inventors also found that at the time of preparing a slurry to be provided for such drying, when a powder component and a non-volatile oil component containing a particular amino-modified silicone are used to prepare a slurry in a volatile solvent, a powdery cosmetic material which is excellent in terms of smoothness when applied on the skin, uniform finish, and spreadability, has excellent adhesion to the skin and an excellent fit feeling, and provides an excellent long-lasting makeup effect, is obtained. Furthermore, the inventors found that when a lipophilic nonionic surfactant is incorporated into the oily component, the powdery cosmetic material has a superior moist feeling.

According to the present invention, production has been made possible of a powdery cosmetic material which is excellent not only in terms of these product qualities, but also in terms of productivity and work environment properties. In addition, when molding of this powdery composition is carried out by conventional dry molding, production has been made possible of a solid powdery cosmetic material which is also very excellent in terms of usage properties such as easy appliability with a puff.

A suitable embodiment of the present invention will be described with reference to the attached drawings.

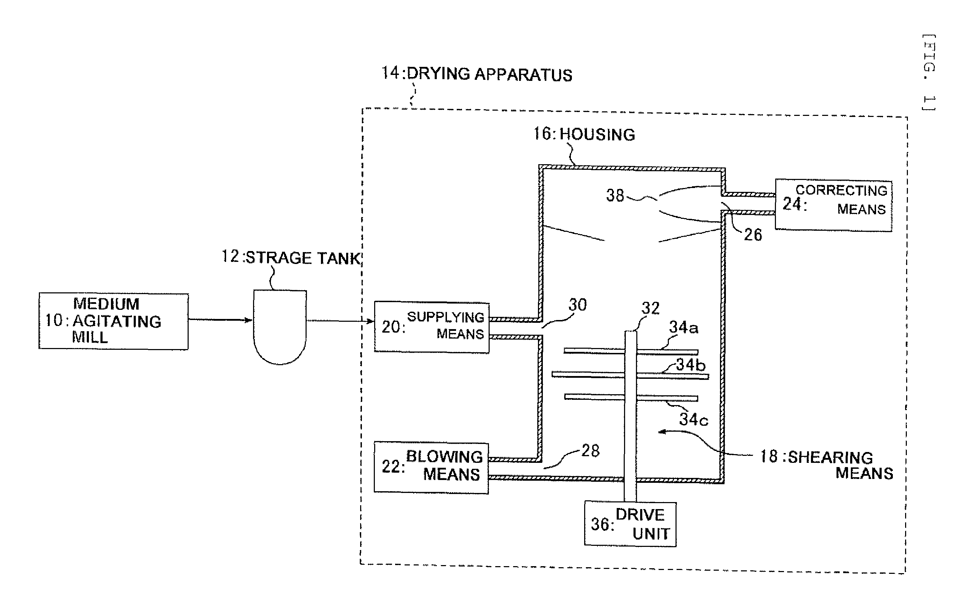

FIG. 1 is a diagram showing an example of the configuration of the apparatus used in the production method of the present invention. The production method of the present invention includes a slurry preparation step of mixing a powder component and a non-volatile oily component in a volatile solvent to obtain a slurry, and a drying step of drying the slurry to remove the volatile solvent, and obtaining a powdery composition.
First, in the slurry preparation step, a slurry is obtained by mixing the powder component and the non-volatile oily component in the volatile solvent using the medium agitating mill 10 shown in FIG. 1, and cracking, pulverizing, and dispersing the powder component (slurry preparation step) . The slurry thus obtained is temporarily collected in a storage tank 12, and is supplied to a drying apparatus 14 (drying step) at a predetermined flow rate.

The drying apparatus 14 used in the present embodiment performs drying of the slurry by converting the slurry into fine liquid droplets through a mechanical shear force, that is, a shear force generated by the rotation of shearing members (plate-shaped members 34a, 34b and 34c) provided in a shearing means 18, and blowing a dry gas to the fine liquid droplets. There are no particular limitations on the shape of the shearing members as long as the shape is adequate for the purpose, and for example, the shearing members may have any shape such as a blade shape or a disc shape, in addition to the plate shape such as described above.

As such, in the present embodiment, since a powdery composition is produced using a drying apparatus 14 which performs drying of the slurry in the form of fine liquid droplets, a powdery composition can be obtained almost without the occurrence of aggregation of the powder component at the time of drying. Accordingly, it is possible to provide a powdery cosmetic material having an excellent feeling of use when applied on the skin. Also, because it is not necessary to carry out cracking again after drying, the production of the powdery composition is also excellent in terms of productivity and work environment properties.

It is also suitable to further include a step of filling the powdery composition thus obtained in a container and solidifying the powdery composition by dry press molding. The solid powdery cosmetic material thus obtained not only has an excellent feeling of use, but also has excellent usage properties such as easy appliability with a puff.

As shown in FIG. 1, in the slurry preparation step, it is suitable to use a medium agitating mill 10 in order to mix the essential components to be incorporated in a volatile solvent. When a medium agitating mill is used, a slurry in which the powder component is coated on the surface with the non-volatile oily component appropriately uniformly to the extent that will not affect the long-lasting makeup effect, can be obtained. When such a slurry is used, a powdery cosmetic material which has a superior feeling of use and superior usage properties can be obtained.

Furthermore, when a powdery cosmetic material containing a pearl pigment is produced, first, a slurry is prepared using a powder component except for the pearl pigment in the slurry preparation step, and this slurry is dried in the drying step to obtain a powdery composition which does not contain the pearl pigment. Then, the powdery composition which does not contain the pearl pigment is mixed with the pearl pigment, and a powdery cosmetic material is suitably obtained using this powder mixture. When such a production method is employed, there can be obtained a powdery cosmetic material which is excellent in terms of product quality, such as smoothness, a feeling of fit to the skin, uniform finish, a moist feeling, and good spreadability, and in terms of productivity and work environment properties, and also has an excellent pearl-like texture.

Hereinafter, the respective steps will be described in more detail.

### <Slurry preparation step>

As the method of mixing a powdery component and a non-volatile oily component in a volatile solvent to obtain a slurry, methods such as described below may be used.
(A) A method of adding a product which has been prepared by dry mixing/cracking a powder component and a non-volatile oily component in advance with a Henschel mixer (registered trademark), a pulverizer or the like, into a volatile solvent, and mixing/dispersing the components using a disper-mixer, a homogenizer, a planetary mixer, a biaxial kneading machine or the like.
(B) A method of adding a powder component and a non-volatile oily component into a volatile solvent, preliminarily mixing the components with the solvent with a disper-mixer or the like as necessary, and then subjecting the mixture to cracking, pulverization and dispersion using a medium agitating mill.
(C) A method of adding a certain specific powder component having strong aggregability, such as an elastic polymer powder or a fine particle powder, into a volatile solvent, preliminarily mixing this powder component with the solvent with a disper-mixer or the like as necessary, subsequently cracking, pulverizing and dispersing the mixture using a medium agitating mill to obtain a dispersion liquid, adding another powder component or a non-volatile oily component to the dispersion liquid, and further processing the mixture using a wet mixing machine or a medium agitating mill.

In the slurry preparation step, it is suitable to use a medium agitating mill {for example, the methods (B) and (C)}. A medium agitating mill is a device which accommodates a dispersion liquid formed from a powder component (and a non-volatile oily component) and a solvent in a vessel containing a solid dispersing medium (medium) such as beads, and agitating the liquid in the vessel to perform cracking, pulverization and dispersion of the powder component by means of an impact force, a frictional force or the like exerted by the medium.

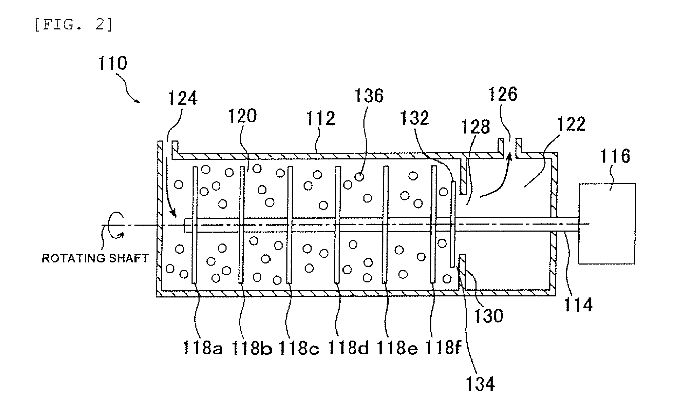

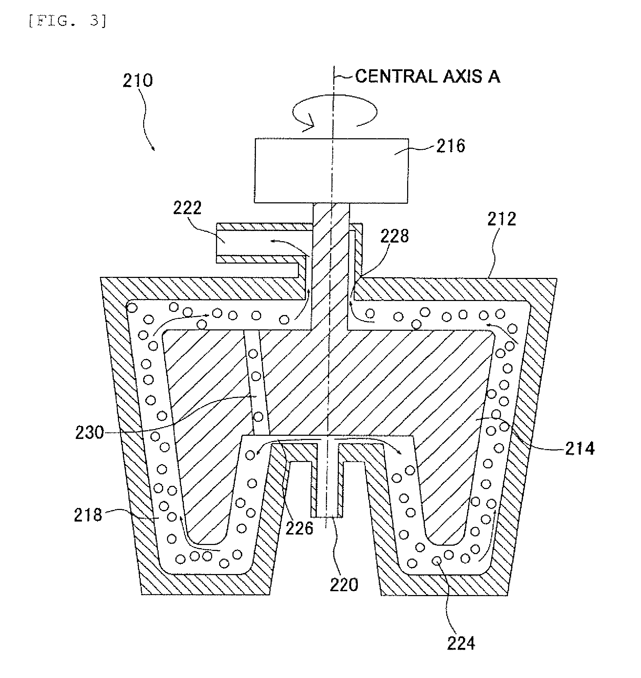

FIG. 2 and FIG. 3 are schematic configuration diagrams respectively showing examples of the medium agitating mill that is suitably used in the present invention. The medium agitating mills that can be suitably used in the present invention are not limited to these, and any medium agitating mill may be used as long as it is capable of achieving the object of the present invention.

The medium agitating mill 110 shown in FIG. 2 as an example includes an generally cylindrical-shaped vessel 112, a drive shaft 114 inserted through the vessel 112, a drive motor 116 which rotationally drives the drive shaft 114, and plural sheets of agitating discs 118a to 118f that are fitted to the drive shaft. The inside of the vessel 112 is divided into a dispersing chamber 120 which performs cracking, pulverization and dispersion of the powder component, and a extraction chamber 122 which extracts the processed dispersion liquid. The dispersing chamber 120 of the vessel 112 is provided with a supply port 124 for supplying a dispersion liquid to be processed, and the extraction chamber 122 is provided with a extraction port 126 for extracting the processed dispersion liquid. A partition wall 130 provided with an opening 128 is provided between the dispersing chamber 120 and the extraction chamber 122, and near this partition wall 130, a separating disc 132 fitted to the drive shaft 114 is disposed to cover the opening 128 of the partition wall 130. A gap is provided between the partition wall 130 and the separating disc 132, and this gap is used as a separating slit 134 which separates the solid dispersing medium from the dispersion liquid to be processed.

The dispersion liquid containing the powder component, the non-volatile oily component, and the volatile solvent (hereinafter, simply referred to as solvent) is successively supplied through the supply port 124 to the dispersing chamber 120 inside the vessel 112, and the dispersion liquid in the dispersing chamber 120 moves successively in the direction of the extraction chamber 122. At this time, the drive shaft 114 is rotationally driven by the drive motor 116, and the agitating discs 118a to 118f are rotating. The dispersing chamber 120 is filled with a large number of solid dispersing media 136, and the solid dispersing media 136 are agitated together with the dispersion liquid by the rotation of the agitating discs 118a to 118f. The aggregated powder component in the dispersion liquid is cracked, pulverized and dispersed by the impact force, shear stress or the like exerted by the solid dispersing media 136.
The dispersion liquid that has been subjected to cracking, pulverization and dispersion as described above, passes through the separating slit 134 that is located between the partition wall 130 between the dispersing chamber 120 and the extraction chamber 122 and the separating disc 132, flows into the extraction chamber 122 and is extracted to the outside through the extraction port 126. The separating slit 134 has a size that will prevent the solid dispersing media 136 from escaping from the dispersing chamber 120 to the extraction chamber 122. Accordingly, when the dispersion liquid passes through the separating slit 134, the separating slit separates the dispersion liquid (powder component + solvent) from the solid dispersing media 136, so that only the dispersion liquid enters the extraction chamber.

FIG. 3 is a schematic configuration diagram of an annular type medium agitating mill. The medium agitating mill 210 of FIG. 3 includes a vessel 212 which has an generally W-shaped cross-section that is symmetric about the central axis A, a rotor 214 which is generally inversely U-shaped and is capable of rotating around the central axis A, and a drive motor 216 that rotationally drives the rotor 214. A circular space 218 is formed between the inner surface of the vessel 216 and the outer surface of the rotor 214, and this circular space 218 has a shape having an generally V-shaped cross-section which lies on both sides of the central axis A. Furthermore, a supply port 220 for sending the dispersion liquid (powder component + solvent) to be processed to the circular space 218, and a extraction port 222 for extracting the dispersion liquid to be processed from the circular space 218, are formed in the vessel 212. The circular space 218 is filled with solid dispersing media 224, so that the circular space 218 is used as a dispersing chamber that performs cracking, pulverization and dispersion of the powder component in the dispersion liquid.

The dispersion liquid supplied from the supply port 220 is transported to the circular space 218 via an inlet slit 226. The dispersion liquid thus transported moves inside the circular space 218 and is extracted through the extraction port 222 via an outlet slit 228. At this time, the dispersion liquid and the solid dispersion media 224 inside the circular space 218 are agitated by rotating the rotor 214 about the central axis A within the circular space 218. Then, the aggregated powder component in the dispersion liquid is cracked, pulverized and dispersed by the impact force, shear stress or the like exerted from the solid dispersing media 224. Thereafter, the dispersion liquid passes through the outlet slit 228 and is extracted from the extraction port 222.
The outlet slit 228 has a size that will prevent the solid dispersing media 224 from escaping from the circular space 218, and functions as a separating means that separates the dispersion liquid (powder component + solvent) from the solid dispersing media 224. Furthermore, the rotor 214 is provided with a returning hole 230 for returning the solid dispersing media 224 to the inlet side, so that the solid dispersing media 224 are kept from staying in the vicinity of the outlet.

The reason for cracking, pulverizing and dispersing a powder component and a non-volatile oily component in a volatile solvent using a medium agitating mill is that since the mixed and dispersed state of the powder component and the non-volatile oily component can be enhanced, and the surface of the powder component can be coated with the non-volatile oily component appropriately uniformly to the extent that will not affect the long-lasting makeup effect, a powdery cosmetic material having a good feeling of use can be obtained. Furthermore, a powder having strong aggregability can be easily cracked and uniformly dispersed in a volatile solvent.

Examples of the medium agitating mill have been described above, but in addition to those, suitable examples include batch type bead mills such as a basket mill; horizontal type, vertical type, and annular type continuous bead mills; sand grinder mills; ball mills; Micros (registered trademark) and the like. However, there are no particular limitations on the medium agitating mill as long as the medium agitating mill adequate for the purpose. That is, any medium agitating mill which, when a powder component which is in an aggregated state is incorporated, can resolve the aggregation of the powder component to agitate and disperse the powder component to a state close to primary particles, and can appropriately uniformly coat the powder surfaces with a non-volatile oily component to the extent that will not affect the long-lasting makeup effect, can be used without any particular limitations.

The media to be used in the medium agitating mill are preferably beads, and beads produced from raw materials such as glass, alumina, zirconia, steel and flint may be used, while beads made of zirconia are particularly preferred. In regard to the size of the beads, usually beads having a diameter of about 0.5 to 10 mm are used with preference, but in the present invention, beads having a diameter of approximately 2 mm to 5 mm are used with preference. If the size of the bead diameter is too small, cracking of the extender pigments such as mica and talc proceeds excessively, and an adverse effect is exerted on the feeling of use, or the hardness after molding is increased so that appliability is deteriorated, or caking or the like is easily induced. On the other hand, if the size of the bead is too large, aggregation of the powder component cannot be sufficiently resolved, and uniform coating with a non-volatile oily component becomes difficult.

The powder component and the non-volatile oily component that are mixed in the slurry preparation step will be described below. Furthermore, components that can be incorporated into the powder cosmetic material in addition to the essential components in the slurry preparation step will also be described below.

There are no particular limitations on the volatile solvent that is used to mix the incorporated components of the powder cosmetic material, but examples of the volatile solvent include purified water, cyclic silicones, ethanol, light liquid isoparaffin, lower alcohols, ethers, LPG, fluorocarbons, N-methylpyrrolidone, fluoroalcohols, volatile linear silicones, and next generation Freon. Representative examples of the lower alcohols include ethanol and isopropanol. These solvents are used properly with different purposes, singly or as mixtures of two or more kinds, in accordance with the characteristics of the incorporated components that are used.

The amount of the volatile solvent that is used in the slurry preparation step cannot be specified because the amount depends on the polarity, specific gravity and the like of the volatile solvent used, but in the case of using a medium agitating mill, it is important to secure fluidity that enables the process.

### <Drying step>

Next, an example of the drying apparatus that is used in the drying step of an embodiment according to the present invention will be described, with reference to FIG. 1. The drying apparatus used in the production method of the present invention is not limited to that in FIG. 1, and any drying apparatus equipped with a shearing means which mechanically converts a slurry into fine liquid droplets is acceptable. The drying apparatus 14 in FIG. 1 includes a hollow-shaped housing 16 which serves as the site for performing drying of the slurry; a shearing means 18 which converts a slurry into fine liquid droplets by means of rotating shearing members (plate-shaped members 34a, 34b, and 34c) that are provided inside the housing 16; a supplying means 20 which supplies the slurry to the shearing members (plate-shaped members 34a, 34b and 34c) inside the housing 16; a blowing means 22 which blows a dry gas into the housing 16, and supplies the dry gas to the slurry that has been converted to fine liquid droplets by the shearing means 18; and a collecting means 24 which collects a powdery composition generated by drying the slurry.

The housing 16 has an generally cylindrical shape which is vertical and hollow, and is provided at the upper part with an emission port 26 which emits the powdery composition and the dry gas, and is provided at the lower part with a blowing port 28 which supplies the dry gas from the blowing means 22 into the housing 16. Also, a supply port 30 which supplies the slurry into the housing 16 is disposed between the emission port 26 located at the upper part of the housing 16 and the blowing port 28 located at the lower part.
The shearing means 18 includes a rotating shaft 32 that is installed in the direction perpendicular to the bottom of the housing 16, shearing members (plate-shaped members 34a, 34b and 34c) that are provided at a right angle to the rotating shaft 32, and a drive means 36 for rotating the rotating shaft 32. The drive means 36 is disposed outside the housing 16, and transfers a rotating force to the shearing members (plate-shaped members 34a, 34b and 34c) through the rotating shaft 32. The shearing members shown in FIG. 1 are constituted by three plate-shaped members 34a, 34b and 34c that are provided at a right angle to the rotating shaft 32 at intervals in the vertical direction. These shearing members are located in the lower part of the supply port 30 for the slurry and in the upper part of the blowing port 28 for the dry gas. When the rotating shaft 32 is rotated by the drive means 36 composed of a motor and the like, the plate-shaped members 34a, 34b and 34c rotate in the horizontal direction about the rotating shaft 32 inside the housing 16, and this mechanical shear force causes the conversion of the slurry into fine liquid droplets.

The supply means 20 supplies the slurry sent from the storage tank 12 into the housing 16. The slurry supplied to the housing 16 drops toward the plate-shaped members 34a, 34b and 34c, and is converted into fine liquid droplets by the rotating plate-shaped members 34a, 34b and 34c. Furthermore, the dry gas sent from the blowing means 22 is blown into the housing 16 through the blowing port 28. The dry gas is supplied toward the tangential direction of the horizontal cross-section of the housing 16, and since the plate-shaped members 34a, 34b and 34c are in a rotating movement, the dry gas stream blown into the housing 16 is changed to a swirling flow. When the slurry in the form of fine liquid droplets is brought into contact with this dry gas stream, the slurry is further micronized and is dried to form a powdery composition. This powdery composition is blown up together with the dry gas stream to the upper part of the housing 16, and is emitted through the emission port 26. The powdery composition emitted out of the housing 16 through the emission port 26 is collected by a collection means 24.
Furthermore, a classification means 38 is provided in the area of the emission port 26 inside the housing 16. The classification means 38 is constructed as an orifice provided at the emission port 26, and prevents large grains, lumps, undried materials and the like from entering the collection means 24. The configuration of the classification means is not limited to this, and any other configuration may be used.

As such, when a mechanical shear force is exerted on the slurry by the shearing members (plate-shaped members 34a, 34b and 34c), and the slurry is converted to fine liquid droplets and dried, a powdery composition with less aggregation can be obtained. The reason why a powdery composition with less aggregation is obtained is speculated to be that when the slurry is converted to fine liquid droplets, the amount of the powder component present in the liquid droplets is small, and therefore, aggregation at the time of drying does not easily occur, that the aggregation of the powder component occurring during the drying process is resolved by the shear force exerted by the shearing members or the swirling flow, and the like.
An example of using shearing members constituted of plate-shaped members that rotate in the horizontal direction has been described herein, but in addition to this, shearing members constituted of plate-shaped members that rotate in the vertical direction (the rotating shaft extending in the horizontal direction) may also be provided. Furthermore, the shape of the shearing member is not limited to the shape described above, and for example, a blade shape (a cutter provided perpendicularly to the tip of a rod-shaped member which is attached perpendicularly to the rotating shaft, or the like), a disc shape and the like may be used. Also, there are no particular limitations on the number of the shearing members, or the like.

The drying apparatus described above is of a type called flash dryer, and examples thereof include a Spin Flash Dryer manufactured by APV Nordic Anhyro A/S, a Drymeister manufactured by Hosokawa Micron Corp., and a vertical agitation dryer manufactured by Tsukishima Kikai Co., Ltd. There are no limitations on the drying apparatus that may be suitably used in the present invention, and any of vertical type and horizontal type drying apparatuses may be used as long as the apparatus has a shearing mechanism in the system.
The temperature of the dry gas used at the time of drying can be varied depending on the boiling point of the volatile solvent used. Also, since the drying efficiency increases as the temperature of the dry gas is higher, it is desirable to set the temperature of the dry gas high, to the extent that there are no adverse effects such as heat-induced alteration of the constituent components of the powdery composition.
Furthermore, when an inert gas such as nitrogen gas or Ar gas is included in the housing 16, the apparatus acquires excellent explosion proofness, and therefore, the work environment properties are also improved. In addition, recovery of the solvent is also made possible by adopting a solvent recovery mechanism such as a condenser.

### <Solidification step>

When a solid powdery cosmetic material is prepared using the powdery composition obtained by the production method of the present invention, it is suitable that the production method further includes a solidification step of filling the powdery composition in a container and solidifying the powdery composition by dry molding. As the method for solidification, conventionally known dry press molding and the like may be used. A solid powdery cosmetic material thus obtained exhibits an excellent feeling of use that is equivalent to or superior than the solid powdery cosmetic materials produced by wet methods, and also has an advantage of good usage properties (appliability with a puff), which is an advantage of dry molding. Furthermore, in the case of conventional wet molding methods including a step of filling a slurry in a container by injection filling, since it is necessary to take fillability of the slurry into consideration, there are limitations on the raw materials to be used. However, it is an advantage of the production method of the present invention that as long as conventional dry press molding is carried out, there are no limitations on the raw materials to be used.

### <In case of incorporating pearl pigment>

In the case of producing a powdery cosmetic material containing an added pearl pigment, representative examples of which include titanated mica, glass pearl and the like, when the production is carried out using a medium agitating mill, it is preferable to first obtain a powdery composition using the portion of the powder component excluding the pearl pigment, by subjecting the powder component to the slurry preparation step and the drying step described above. This powdery composition and a necessary amount of a pearl pigment are mixed in a dry mixing machine which exerts a weak shear force, such as a Henschel mixer or a Nauta mixer, to obtain a mixed powder, and this mixed powder is filled in a container and is further subjected to dry molding to obtain a powdery cosmetic material. The powdery cosmetic material obtained by this method is excellent in terms of the feeling of use, usage properties, and the long-lasting makeup effect, and also has an excellent pearl-like texture.

There are no particular limitations on the pearl pigment, and those pearl pigments that are generally used as conventional cosmetic materials can be used. Representative examples thereof include titanated mica, iron oxide-coated titanated mica, lower-order titanium oxide-coated titanated mica, photochromic titanated mica, and pigments which use talc, glass, synthetic fluorphlogopite, silica, bismuth oxychloride and the like instead of mica as substrate. Also, examples of the coating material include, in addition to titanium oxide, lower-order titanium oxide, iron oxide, alumina, silica, zirconia, zinc oxide, cobalt oxide, aluminum and the like. Examples of functional pearl pigments having excellent optical properties include pearl pigments coated on the surfaces with resin particles (JP-A No. 11-92688), pearl pigments coated on the surfaces with aluminum hydroxide particles (JP-A No. 2002-146238), pearl pigments coated on the surfaces with zinc oxide particles (JP-A No. 2003-261421), pearl pigments coated on the surfaces with barium sulfate particles (JP-A No. 2003-61229) and the like. These functional pearl pigments having the pearl pigment surfaces coated with various particles, are such that when a strong force is exerted on the particles that are coating the surfaces, the particles tend to easily fall off. However, when the method described above is used, a force is exerted on the pearl pigment only at the time of mixing with the powdery composition, and therefore, it is not necessary to mix the pigment with the powdery composition with such a strong force, and the particles coating the pearl pigments are less likely to fall off. Therefore, according to the production method described above, a functional pearl pigment can be incorporated into the powdery cosmetic material without impairing the function of the pearl pigment.
The amount of incorporation of the pearl pigment is preferably 0% to 20% by mass relative to the total amount of the powdery cosmetic material.

In the case of producing a powdery cosmetic material, the amount of incorporation of the powdery composition obtained by the production method of the present invention into the cosmetic material is preferably 80% to 100% by mass, and more preferably 90% to 100% by mass, relative to the total amount of the powdery cosmetic material. If a pearl pigment is not incorporated, the powdery composition may be incorporated at a proportion of 100%.

The powdery composition obtained by the production method of the present invention has the non-volatile oily component as a binder and the powder component sufficiently mixed and pulverized, and aggregation occurs less during drying. Therefore, a subsequent pulverization step is not needed, and a solid powdery cosmetic material can be easily produced by directly dry press molding the powdery composition, without adding a non-volatile oily component as an additional binder. Furthermore, when the powdery composition is produced, without press molding, into a powdery cosmetic material such as loose powder, the powdery composition may be directly filled in a container and prepared as a final product.
Furthermore, in the case of preparing products of plural colors, powdery compositions respectively having different compositions may be prepared, and those powdery compositions may be mixed to adjust the color or the like for use.

The powdery composition obtained by the production method of the present invention are suitably applied to powdery cosmetic materials or solid powdery cosmetic materials, such as foundation, eye shadow, rouge, body powder, powdered perfume, baby powder, pressed powder, deodorant powder, and face powder.
Particularly, when used as a solid powdery cosmetic material, the powdery composition exhibits an effect of improving the feeling of use or the long-lasting makeup effect.

Next, the incorporation components of the powdery composition obtained by the production method of the present invention will be described.

### <Powdery component>

The powder that can be incorporated as the powder component used in the slurry preparation step and the drying step of the present invention is not particularly limited, as long as the powder can be used as a cosmetic material. Furthermore, a powder which has been hydrophobized in advance may also be used. According to the production method of the present invention, when a particular amino-modified silicone is contained in the non-volatile oily component, water-repellent and oil-repellent effects can be imparted to the powder. Therefore, even if a powder which has not been hydrophobized in advance is used, a powdery cosmetic material having an excellent long-lasting makeup effect can be obtained.

Specific components of the powder include an extender pigment, a white pigment, a color pigment, a spherical powder and the like. In the method for producing a powder composition of the present invention, plural different powder components such as an extender pigment, a white pigment, and a color pigment can be simultaneously incorporated.
Examples of the extender pigment include talc, kaolin, sericite, muscovite, phlogopite, lepidolite, biotite, calcined talc, calcined sericite, calcined muscovite, calcined phlogopite, vermiculite, magnesium carbonate, calcium carbonate, aluminum silicate, barium silicate, calcium silicate, magnesium silicate, strontium silicate, tungstic acid metal salts, magnesium, silica, zeolite, barium sulfate, calcined calcium sulfate (calcined gypsum), calcium phosphate, fluorapatite, ceramic powder, metal soaps (for example, zinc myristate, calcium palmitate, and aluminum stearate), and red synthetic fluorphlogopite containing iron atoms in the crystal structure.

Among these, the red synthetic fluorphlogopite containing iron atoms in the crystal structure is known as a cosmetic raw material which can impart a vivid color tone and a transparent texture, and has a high quality feeling of use (JP-A No. 6-9210, and JP-A No. 11-279432). However, it is a raw material which undergoes significant dulling of color when wetted with a non-volatile oily component or sebum, and it has been difficult to incorporate the material in large amounts in the conventional art. However, when the production method of the present invention is used, dulling of color can be mitigated.

When the red synthetic fluorphlogopite is incorporated, the amount is preferably 2% to 20% by mass relative to the total amount of the powdery composition after dried. If the amount is less than 2% by mass, there is a possibility that the effect of high quality feeling of use of the red synthetic fluorphlogopite can be hardly expected. On the other hand, if the amount is greater than 20% by mass, there is a possibility that the color change caused by sebum is increased, and the long-lasting makeup effect may be decreased, which is not preferable.

Examples of the white pigment include boron nitride, titanium dioxide, zinc oxide, photochromic titanium oxide (titanium dioxide sintered with iron oxide), and reduced zinc oxide.

Examples of the color pigment include inorganic red pigments (for example, iron oxide (red iron oxide), and iron titanate), inorganic brown pigments (for example, γ-iron oxide), inorganic yellow pigments (for example, yellow iron oxide and yellow clay), inorganic black pigments (for example, black iron oxide and lower-order titanium oxide), inorganic violet pigments (for example, mango violet and cobalt violet), inorganic green pigments (for example, chromium oxide, chromium hydroxide, and cobalt titanate), inorganic blue pigments (for example, ultramarine blue and Prussian blue), organic pigments such as barium or aluminum lakes (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1), and natural coloring materials (for example, chlorophyll and β-carotene).

Examples of the spherical powder include silicone elastomer powder, silicone powder, silicone resin-coated silicone elastomer powder, polyamide resin powder (nylon powder), polyethylene powder, polymethyl methacrylate powder, polystyrene powder, powder of a copolymer resin of styrene and acrylic acid, benzoguanamine resin powder, polyurethane resin powder, and spherical silica.

In addition to those, a powder component that can be conventionally incorporated into cosmetic materials can be incorporated. According to the present invention, one kind or two or more kinds of these powder components, preferably three or more kinds, and more preferably four or more kinds of powder components can be used.

### <Amount of incorporation of powder component>

The amount of incorporation of the powder component used in the slurry preparation step is 70% to 98% by mass, preferably 80% to 95% by mass, and more preferably 85% to 95% by mass, relative to the total amount of the powdery composition after dried. If the amount of incorporation is less than 70% by mass, there is a possibility that the water repellency and oil repellency may be insufficient. Furthermore, if the amount of incorporation is greater than 98% by mass, there is a possibility that the moist feeling may be insufficient.
Particularly, in the case of producing a powdery composition intended to be used in a solid powdery cosmetic material such as powdery foundation, the amount of incorporation of the powder component used in the slurry preparation step is most preferably 85% to 95% by mass relative to the total amount of the powdery composition after dried, from the viewpoint of the balance between the long-lasting makeup effect and the feeling of use such as moist feeling.

### <Non-volatile oily component>

### (a) Amino-modified silicone

According to the present invention, it is essential that the non-volatile oily component contain a particular amino-modified silicone. As the amino-modified silicone used in the present invention, an amino-modified silicone having a functional group equivalent of 1000 to 10,000 and a viscosity at 25°C of 200 to 2000 mm²/s is used.
If the viscosity is less than 200 mm²/s, it is difficult to obtain a sufficient water-repellent and oil-repellent effects, and if the viscosity is greater than 2000 mm²/s, since the fluidity of the oily component is low at the time of drying, aggregation or non-homogenization is prone to occur, and there is a possibility that stable mass production may be hindered.
If the functional group equivalent is less than 1000 or greater than 10,000, the water-repellent and oil-repellent effects may be insufficient.
More preferably, an amino-modified silicone having a functional group equivalent of 1000 to 3000 and a viscosity at 25°C of 600 to 1300 mm²/s is used.

An example of such an amino-modified silicone may be a side-chain diamine type amino-modified silicone represented by the following formula (1). 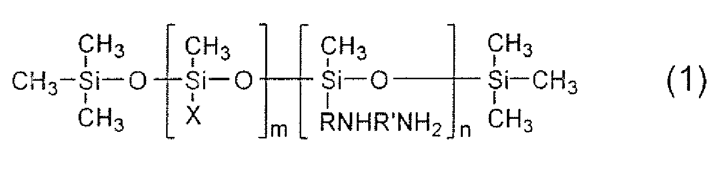 wherein in the formula (1), X, R and R' each represent an alkyl group having 1 to 4 carbon atoms; and m and n each represent a positive number of 1 or greater, so that 1 < m + n < 1000.
A representative compound thereof is known as Amodimethicone (aminoethylaminopropylmethylsiloxane-dimethylsiloxane copolymer), and commercially available products include KF-8002, KF-8004, KF-880, KF-867 (all manufactured by Shin-Etsu Chemical Co., Ltd.) and the like.

The amount of incorporation of the amino-modified silicone used in the present invention is preferably 0.1% to 2% by mass relative to the total amount of the powdery composition after dried. If the amount of incorporation is less than 0.1% by mass, sufficient water-repellent and oil-repellent effects may not be obtained. Furthermore, if the amount of incorporation is greater than 2% by mass, water-repellent and oil-repellent effects may be obtained, but the feeling of use may be deteriorated, which is not preferable.

### (b) Lipophilic nonionic surfactant

According to the present invention, the non-volatile oily component preferably contains a lipophilic nonionic surfactant. As the lipophilic nonionic surfactant, a nonionic surfactant having an HLB value of 2 to 10, and preferably 3 to 6, can be used. Examples thereof include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate); glycerin polyglycerin fatty acids (for example, mono-cottonseed oil fatty acid glycerin, glyceryl monoerucate, glyceryl sesquioleate, glyceryl monostearate, α,α'-glyceryl oleate pyroglutamate, and glyceryl monostearate malate); propylene glycol fatty acid esters (for example, propylene glycol monostearate) ; hardened castor oil derivatives; and glycerin alkyl ethers. Preferably, sorbitan fatty acid esters, sorbitan sesquiisostearate, and sorbitan monoisostearate are used.

The amount of incorporation of the lipophilic nonionic surfactant is preferably 0.1% to 2% by mass relative to the total amount of the powdery composition after dried. If the amount of incorporation is less than 0.1% by mass, the effect of enhancing the moist feeling of use may not be sufficiently obtained. Furthermore, if the amount of incorporation is greater than 2% by mass, a moist feeling may be obtained, but since the powdery composition is likely to be blended with sebum, the long-lasting makeup effect may be deteriorated, which is not preferable.

In addition to those, examples of the non-volatile oily component used in the present invention include, in addition to the particular amino-modified silicone and the lipophilic nonionic surfactants, liquid fats and oils, solid fats and oils, waxes, hydrocarbons, higher fatty acids, higher alcohols, ester oils, silicone oils, oil-soluble ultraviolet absorbers such as octocrylene and octyl methoxycinnamate and the like, and there are no particular limitations as long as the oily component can be used in cosmetic materials. The non-volatile oily components can be used singly or in combination of two or more kinds. The non-volatile oily component can function as a binder for solid powdery cosmetic materials.

The amount of incorporation of the non-volatile oily component is 2% to 30% by mass, preferably 5% to 20% by mass, and more preferably 5% to 15% by mass, relative to the total amount of the powdery composition after dried. If the amount of the non-volatile oily component is too small, moldability for the solidification step, and the feeling of use such as smoothness, a feeling of fit to the skin, uniform finish, a moist feeling, and spreadability may be insufficient. On the other hand, if the amount of the oily component is too large, the feeling of use as a powdery cosmetic material, such as stickiness, may be impaired, or aggregation may occur during the drying step.

### <Other incorporable components>

According to the production method of the present invention, in addition to the essential components described above, other components that are conventionally used in cosmetic materials, for example, moisturizers such as polyhydric alcohols, sugars, and amino acids; water-soluble polymers, thickeners, film-forming agents, ultraviolet absorbers, metal ion sequestering agents, pH adjusting agents, skin nutriments, vitamins, various plant extracts, other efficacious ingredients, antioxidants, antiseptics, and fragrances can be appropriately incorporated as necessary, to the extent that the effects of the present invention are not impaired, and cosmetic materials can be produced by conventional methods in accordance with intended products. These components are usually mixed with the essential components during the slurry preparation step, but fragrances and the like may be mixed with the powdery composition after the drying step.

### EXAMPLES

Hereinafter, the present invention will be further described by way of Examples, but the present invention is not intended to be limited to these. Unless particularly stated otherwise, the amount of incorporation is expressed in percent (%) by mass.

The method for evaluation of the feeling of use (good spreadability, a feeling of fit to the skin, smoothness, a moist feeling, and fine quality) and the long-lasting makeup effect over time (fading, shining, dulling of color, and powder flaking) of the powdery cosmetic material is as follows.

### <Method for evaluation>

A panel of ten experts for evaluation actually used the cosmetic material and performed a sensory test on various evaluation items. The evaluation results were graded according to the following four criteria.
A: Eight or more out of ten experts considered the cosmetic material to be satisfactory.
B: Six to seven out of ten experts considered the cosmetic material to be satisfactory.
C: Four to five out of ten experts considered the cosmetic material to be satisfactory.
D: Three or fewer out of ten experts considered the cosmetic material to be satisfactory.

In Test Example 1-1 to Test Example 1-6 having the compositions indicated in Table 1, the powder component and the non-volatile oily component were mixed in ethyl alcohol (50% by mass in outparts) using a disper-mixer, and the mixture was cracked, pulverized and dispersed using a medium agitating mill (sand grinder mill, using zirconia beads having a diameter of 2 mmφ). Thus, a slurry was obtained. Subsequently, the slurry was converted into fine liquid droplets by a mechanical shear force using a Spin Flash Dryer (manufactured by APV Nordic Anhyro A/S), and a dry gas was blown to dry the fine liquid droplets. Thus, a powdery composition was obtained. The powdery composition thus obtained was filled in a medium-sized dish, and was subjected to dry press molding by a known method. Thus, a solid powdery cosmetic material was produced.
In Test Example 1-7, the solid powdery cosmetic material was produced using a conventional, so-called dry method. In Test Example 1-7, the powder component and the non-volatile oily component were mixed in a Henschel mixer, and then the mixture was pulverized two times with a pulverizer. Subsequently, the pulverization product was filled in a medium-sized dish, and was subjected to dry press molding by a known method. Thus, a solid powdery cosmetic material was obtained.

In Tables 1 to 3, an aminoethylaminopropylmethylsiloxane-dimethylsiloxane copolymer having a functional group equivalent of 1500 and a viscosity at 25°C of 800 mm²/s was used as the amino-modified silicone (*1). Furthermore, as the highly polymerized, amino-modified silicone (*2), a 20% solution prepared by dissolving a rubber-like, highly polymerized, amino-modified silicone having a functional group equivalent which was greater than 50,000 in dimethylsilicone was used.

**[Table 1]**

| | Test Example 1-1 | Test Example 1-2 | Test Example 1-3 | Test Example 1-4 | Test Example 1-5 | Test Example 1-6 | Test Example 1-7 |
|---|---|---|---|---|---|---|---|
| (Powder component) | | | | | | | |
| Talc | balance | balance | balance | balance | balance | balance | balance |
| Synthetic fluorphlogopite | 15 | 15 | 15 | 15 | 15 | 15 | 15 |
| Sericite | 15 | 15 | 15 | 15 | 15 | 15 | 15 |
| Barium sulfate | 10 | 10 | 10 | 10 | 10 | 10 | 10 |
| Titanium oxide | 15 | 15 | 15 | 15 | 15 | 15 | 15 |
| Iron oxide | 3.2 | 3.2 | 3.2 | 3.2 | 3.2 | 3.2 | 3.2 |
| Organopolysiloxane elastomer spherical powder (average particle size 5 µm) | 5 | 5 | 5 | 5 | 5 | 5 | 5 |
| Spherical PMMA powder | 5 | 5 | 5 | 5 | 5 | 5 | 5 |
| Zinc oxide | 5 | 5 | 5 | 5 | 5 | 5 | 5 |
| (Non-volatile oily component) | | | | | | | |
| Petrolatum | 1 | 1 | 1 | 1 | 1 | 1 | 1 |
| Squalane | 1 | 1 | 1 | 1 | 1 | 1 | 1 |
| Diisostearyl malate | 2 | 2 | 2 | 2 | 2 | 2 | 2 |
| Dimethicone | 2 | 2 | 2 | 2 | 2 | 2 | 2 |
| Octyl methoxycinnamate | 1 | 1 | 1 | 1 | 1 | 1 | 1 |
| Amino-modified silicone (*1) | 0.01 | 3 | 0.1 | 1 | 2 | - | 1 |
| Highly polymerized amino-modified silicone (*2) | - | - | - | - | - | 1 | - |
| Antiseptic | q.s. | q.s. | q.s. | q.s. | q.s. | q.s. | q.s. |
| Antioxidan | q.s. | q.s. | q.s. | q.s. | q.s. | q.s. | q.s. |
| Long-lasting makeup | C | B | B | A | A | B | C |
| Spreadability | C | C | B | B | B | C | C |
| Feeling of fit to skin | C | B | B | B | B | C | C |
| Smoothness | C | C | B | B | B | C | C |

As shown in Table 1, in regard to the particular production method, in Test Example 1-3 to Test Example 1-5, when a particular amino-modified silicone is incorporated, a superior long-lasting makeup effect was confirmed, and also, it was confirmed that spreadability, the fit feeling, and smoothness are enhanced. This is speculated to be because when the slurry is prepared with a medium mill, the surfaces of the powder component are uniformly coated with the oil component and the amino-modified silicone, and this state is maintained even after the powder component is prepared into a powdery composition.
On the other hand, in Test Example 1-7 using a conventional, so-called dry method, even if a particular amino-modified silicone was incorporated, the effect of enhancing the long-lasting makeup effect was insufficient, and the feeling of use was inferior.
Furthermore, when a highly polymerized, amino-modified silicone was used, the long-lasting makeup effect slightly improved, but a satisfactory feeling of use effect was not obtained (Test Example 1-6).
The amount of incorporation of the amino-modified silicone was examined, and as a result, it was found that a suitable amount of incorporation was 0.1% to 2% by mass (Test Examples 1-1 to 1-5).

Next, solid powdery cosmetic materials having the compositions indicated in Table 2 were produced by fixing the amount of incorporation of the amino-modified silicone, and changing the amount of incorporation of sorbitan sesquiisostearate, which is a lipophilic nonionic surfactant. For the production method, the solid powdery cosmetic materials were all produced by the same process as that used in Test Example 1-1. Evaluations of the long-lasting makeup effect and the feeling of use are presented in Table 2.

**[Table 2]**

| | Test Example 2-1 | Test Example 2-2 | Test Example 2-3 | Test Example 2-4 | Test Example 2-5 | Test Example 2-6 | Test Example 2-7 |
|---|---|---|---|---|---|---|---|
| (Powder component) | | | | | | | |
| Talc | blance | balance | balance | balance | balance | balance | balance |
| Synthetic fluorphlogo pite | 15 | 15 | 15 | 15 | 15 | 15 | 15 |
| Sericite | 15 | 15 | 15 | 15 | 15 | 15 | 15 |
| Barium sulfate | 10 | 10 | 10 | 10 | 10 | 10 | 10 |
| Titanium oxide | 15 | 15 | 15 | 15 | 15 | 15 | 15 |
| Iron oxide | 3.2 | 3.2 | 3.2 | 3.2 | 3.2 | 3.2 | 3.2 |
| Organopolys iloxane elastomer spherical powder (average particle size 5 µm) | 5 | 5 | 5 | 5 | 5 | 5 | 5 |
| Spherical PMMA powder | 5 | 5 | 5 | 5 | 5 | 5 | 5 |
| Zinc oxide | 5 | 5 | 5 | 5 | 5 | 5 | 5 |
| (Oily component) | | | | | | | |
| Petrolatum | 1 | 1 | 1 | 1 | 1 | 1 | 1 |
| Squalane 1 | | 1 | 1 | 1 | 1 | 1 | 1 |
| Diisosteary 1 malate | 2 | 2 | 2 | 2 | 2 | 2 | 2 |
| Dimethicone | 2 | 2 | 2 | 2 | 2 | 2 | 2 |
| Octyl methoxycinn amate | 1 | 1 | 1 | 1 | 1 | 1 | 1 |
| Amino-modif ied silicone (*1) | 1 | - | 1 | 1 | 1 | 1 | 1 |
| Sorbitan sesquiisost earate (HLB = 4.8) | - | 1 | 0.01 | 3 | 0.1 | 1 | 2 |
| Antiseptic | q.s. | q.s. | q.s. | q.s. | q.s. | q.s. | q.s. |
| Antioxidant | q.s. | q.s. | q.s. | q.s. | q.s. | q.s. | q.s. |
| Long-lastin g makeup | A | C | A | C | A | A | B |
| Smoothness | B | B | B | B | B | B | B |
| Moist feeling | C | B | C | A | B | A | A |

As shown in Table 2, in Test Examples 2-5 to 2-7, it was confirmed that when sorbitan sesquiisostearate, a lipophilic nonionic surfactant, was used in combination, the moist feeling was superiorly enhanced, while the long-lasting makeup effect was maintained. On the other hand, as shown in Test Example 2-2, it was confirmed that when only sorbitan sesquiisostearate was incorporated, the usage properties were excellent, but the water-repellent and oil-repellent effects were not obtained, and therefore, the long-lasting makeup effect was deteriorated. Also, in Test Example 2-3, the effect of sorbitan sesquiisostearate of improving the usage properties was not obtained, and in Test Example 2-4, it was confirmed that although the moist feeling was enhanced, the cosmetic material was prone to be blended with sebum, and therefore, the cosmetic material lacked the long-lasting makeup effect. As discussed above, in regard to the production method of the present invention, it was found that when a lipophilic nonionic surfactant was incorporated in an amount of 0.1% to 2% by mass in addition to the particular amino-modified silicone as an oily component, the long-lasting makeup effect and the moist feeling were excellent.

Furthermore, solid powdery cosmetic materials having the compositions indicated in Table 3 were produced by fixing the amounts of incorporation of the amino-modified silicone and sorbitan sesquiisostearate, and changing the amount of incorporation of red synthetic fluorphlogopite containing iron atoms in the crystal structure, and an evaluation of the feeling of use was performed. For the production method, the solid powdery cosmetic materials were all produced by the same process as that used in Test Example 1-1.

**[Table 3]**

| | Test Example 3-1 | Test Example 3-2 | Test Example 3-3 | Test Example 3-4 | Test Example 3-5 |
|---|---|---|---|---|---|
| (Powder component) | | | | | |
| Talc | Balance | Balance | Balance | Balance | Balance |
| Synthetic fluorphlogopite | 29.5 | 5 | 28 | 20 | 10 |
| Red synthetic fluorphlogopite | 0.5 | 25 | 2 | 10 | 20 |
| Sericite | 15 | 15 | 15 | 15 | 15 |
| Barium sulfate | 10 | 10 | 10 | 10 | 10 |
| Titanium oxide | 15 | 15 | 15 | 15 | 15 |
| Iron oxide | 3.2 | 3.2 | 3.2 | 3.2 | 3.2 |
| Organopolysiloxane elastomer spherical powder (average particle size 5 µm) | 5 | 5 | 5 | 5 | 5 |
| Spherical PMMA powder | 5 | 5 | 5 | 5 | 5 |
| Zinc oxide | 5 | 5 | 5 | 5 | 5 |
| (Oily component) | | | | | |
| Petrolatum | 1 | 1 | 1 | 1 | 1 |
| Squalane | 1 | 1 | 1 | 1 | 1 |
| Diisostearyl malate | 2 | 2 | 2 | 2 | 2 |
| Dimethicone | 2 | 2 | 2 | 2 | 2 |
| Octyl methoxycinnamate | 1 | 1 | 1 | 1 | 1 |
| Amino-modified silicone (*1) | 1 | 1 | 1 | 1 | 1 |
| Sorbitan sesquiisostearate (HLB = 4.8) | 1 | 1 | 1 | 1 | 1 |
| Antiseptic | q.s. | q.s. | q.s. | q.s. | q.s. |
| Antioxidant | q.s. | q.s. | q.s. | q.s. | q.s. |
| Dulling of color over time | A | C | A | A | B |
| Smoothness | B | A | B | A | A |
| Moist feeling | A | A | A | A | A |
| High quality finish | C | A | B | A | A |

As shown in Table 3, it was confirmed that when red synthetic fluorphlogopite was used in combination in an amount of 2% to 20% by mass, smoothness as well as high quality properties were superiorly enhanced (Test Examples 3-3 to 3-5). On the other hand, if the amount of incorporation was too small as in Test Example 3-1, the effect of red synthetic fluorphlogopite of improving the usage properties was not exhibited. In Test Example 3-2 in which red synthetic fluorphlogopite was incorporated in an amount of greater than 20% by mass, the high quality properties were superiorly enhanced, but a tendency was confirmed that dulling of color over time caused by red fluorphlogopite was conspicuous. As discussed above, according to the production method of the present invention, even if red fluorphlogopite, which has a problem of dulling of color, was incorporated, the color did not easily become dull, and a high quality feeling of finish could be imparted.

### INDUSTRIAL APPLICABILITY

According to the present invention, a production method which improves the product quality of powdery cosmetic materials and ameliorates the productivity for the production process can be provided. Also, a powdery cosmetic material, particularly, a powdery makeup cosmetic material such as foundation, having an excellent moist feeling of use and an excellent long-lasting makeup effect (preventing makeup smudging) can be obtained.

### EXPLANATIONS OF LETTERS OR NUMERALS

- 10: MEDIUM AGITATING MILL
- 12: STORAGE TANK
- 14: DRYING APPARATUS
- 16: HOUSING
- 18: SHEARING MEANS
- 20: SUPPLYING MEANS
- 22: BLOWING MEANS
- 24: COLLECTING MEANS

## Claims

1. A method for producing a powdery composition, the method comprising:
a slurry preparation step of mixing a powder component and a non-volatile oily component in a volatile solvent to obtain a slurry; and
a drying step of drying the slurry to remove the volatile solvent,
wherein in the drying step, a drying apparatus which converts the slurry into fine liquid droplets by a mechanical shear force and blows a dry gas to the fine liquid droplets to perform drying of the slurry, is used, and
the non-volatile oily component contains:
(a) an amino-modified silicone having a functional group equivalent of 1000 to 10,000 and a viscosity at 25°C of 200 to 2000 mm²/s, in an amount of 0.1% to 2% by mass relative to the total amount of the powdery composition, and
(b) a lipophilic nonionic surfactant in an amount of 0. 1% to 2% by mass relative to the total amount of the powdery composition.

2. The method for producing a powdery composition according to claim 1, wherein the drying apparatus used in the drying step comprises:
a hollow-shaped housing;
a shearing means that shears the slurry with a shearing member provided in the housing and converts the slurry into fine liquid droplets;
a supplying means that supplies the slurry to the shearing member inside the housing;
a blowing means that blows a dry gas into the housing, and supplying the drying gas to the slurry that has been converted to the fine liquid droplets by the shearing means to bring the dry gas and the fine liquid droplets into contact; and
a collecting means that collects a powdery composition produced by drying the slurry.

3. The method for producing a powdery composition according to claim 1 or 2, wherein in the slurry preparation step, the powdery component, the oily component, and the processed powder are mixed in the volatile solvent using a medium agitating mill, and the powder component is cracked and/or pulverized and/or dispersed to obtain a slurry.

4. The method for producing a powdery composition according to any one of claims 1 to 3, wherein the powder component is used in an amount of 70% to 98% by mass relative to the total amount of the powdery composition, and the non-volatile oily component is used in an amount of 2% to 30% by mass relative to the total amount of the powdery composition.

5. The method for producing a powdery composition according to any one of claims 1 to 3, wherein the powder component is used in an amount of 80% to 95% by mass relative to the total amount of the powdery composition, and the non-volatile oily component is used in an amount of 5% to 20% by mass relative to the total amount of the powdery composition.

6. The method for producing a powdery composition according to any one of claims 1 to 5, wherein the lipophilic nonionic surfactant includes a sorbitan fatty acid ester.

7. The method for producing a powdery composition according to any one of claims 1 to 6, wherein the powder component further includes an extender pigment, a white pigment, and a color pigment.

8. The method for producing a powdery composition according to any one of claims 1 to 7, wherein the powder component further includes red synthetic fluorphlogopite containing iron atoms in the crystal structure in an amount of 2% to 20% by mass.

9. A powdery cosmetic material comprising the powdery composition obtained by the method according to any one of claims 1 to 8, in an amount of 80% to 100% by mass relative to the total amount of the powdery cosmetic material.

10. A solid powdery cosmetic material obtained by filling the powdery composition obtained by the method according to any one of claims 1 to 8, and other optional components in a container, and solidifying the mixture by dry press molding.

11. A method for producing a solid powdery cosmetic material, the method comprising a solidification step of filling the powdery composition obtained by the method according to any one of claims 1 to 8, and other optional components in a container, and solidifying the mixture by dry press molding.
